# EUROPEAN PATENT APPLICATION

(11) **EP 1 566 141 A1**
(43) Date of publication of application: **24.08.2005**
(21) Application number: 03812292.5
(22) Date of filing: 19.11.2003
(51) Int. Cl.: A61B 1/00

(54) **FLEXIBLE TUBE OF ENDOSCOPE**

(30) Priority: 29.11.2002 JP 2002348741
(71) Applicant: Olympus Corporation, Tokyo 151-0072 (JP)
(72) Inventor: NISHIIE, Takehiro, Hachioji-shi, Tokyo 192-0032 (JP)
(74) Representative: von Hellfeld, Axel
(86) International application number: PCT/JP2003/014710
(87) International publication number: WO 2004/049920

(57) **Abstract**

A flexible tube (15) comprises a mesh tube (38) formed of a mesh of two or more metal wires so as to cover the outer circumference of a spiral tube (37), and an covering tube (39) for covering the outer side of the mesh tube (38). The covering tube (39) is formed using a high polymer material which contracts under heat of autoclave sterilizing. During autoclave sterilizing, force is generated in the flexible tube (15) which attempts to stretch in the longitudinal direction due to the pressure difference between the inside and outside of the endoscope, but this is offset by the force generated in the covering tube (39) which attempts to contract in the longitudinal direction.

## Description

### Technical Field

The present invention relates to a flexible tube for an endoscope, which can be subjected to autoclave sterilization (high-temperature and high-pressure steam sterilization).

### Background Art

Nowadays, endoscopes are widely used in the medical field. The surgeon can insert a fine and elongated inserting portion of the medical endoscope into the body cavity or the like so as to observe deep portions or the like within the body cavity, and furthermore, so as to perform treatment or the like using a treatment tool as necessary. Note that there is the need to disinfect and sterilize the aforementioned medical endoscopes after use in a sure manner.

As a sterilization method for such medical devices, autoclave sterilization (high-temperature and high-pressure steam sterilization) is becoming a mainstream method. The autoclave sterilization (high-temperature and high-pressure steam sterilization) has the advantage of the fact that the devices can be used immediately after sterilization without troublesome operation with low running costs.

As an example of such an endoscope which can be subjected to autoclave sterilization, an arrangement has been proposed as disclosed in Japanese Unexamined Patent Application Publication No. 2000-157484, which includes a check valve that is released (opened) at the time of the pressure within the endoscope becoming equal to or greater than a predetermined value, i.e., becoming high, in order to prevent the pressure within the endoscope from becoming relatively higher than the external pressure.

However, with the endoscope described in the Japanese Unexamined Patent Application Publication No. 2000-157484, in the event that the external portion of the endoscope is pressurized in the pressurizing process in autoclave sterilization, the pressure within the endoscope is lower than the outside thereof, since the pressure within the endoscope is maintained at a low pressure from the check valve being opened in the negative pressure process. This pressure difference within and outside of the endoscope causes the endoscope to be subjected to pressure from the outside of the endoscope toward the inside of the endoscope, over the entirety thereof.

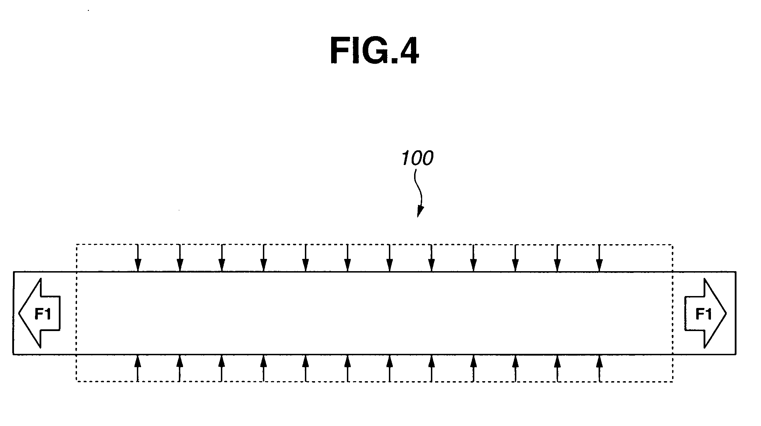

This generates a pressure difference between the inside and outside of the flexible tube 100 of the endoscope insertion portion as shown in the conceptual diagram in Fig. 4, subjecting the flexible tube 100 to a force F1 causing stretching (extension) in the axial direction while reducing the diameter. The force F1 which causes the flexible tube 100 to stretch is such that contracts the diameter of the flexible tube while stretching in the axial direction, by changing the angle of mesh of the mesh tube.

In the event that the flexible tube 100 stretches in this way, there may be trouble such as the components contained therein not being long enough, or the target position becoming offset and the user erroneously judging the length of insertion.

The present invention has been made in light of the above-described point, and it is an object thereof to provide a flexible tube of an endoscope wherein the change in length during autoclave sterilization can be suppressed.

### Disclosure of Invention

With the present invention, a flexible tube of an endoscope has a mesh tube formed by including a mesh of at least two or more metal wires in directions neither orthogonal nor parallel to the longitudinal direction of an insertion portion, wherein a contracting member is provided which generates a contraction force in the longitudinal direction, against and approximately equal to a stretching force in the longitudinal direction which is generated in the event that a pressure difference occurs between the inside and outside of the endoscope.

### Brief Description of the Drawings

Fig. 1 is an overall configuration diagram which illustrates an endoscope apparatus;
Fig. 2 is an explanatory diagram which shows a flexible tube of an inserting portion of the endoscope shown in Fig. 1;
Fig. 3 is an enlarged explanatory diagram which shows a mesh tube forming the flexible tube shown in Fig. 2; and
Fig. 4 is a conceptual diagram which shows force applied to a conventional flexible tube.

### Best Mode for Carrying Out the Invention

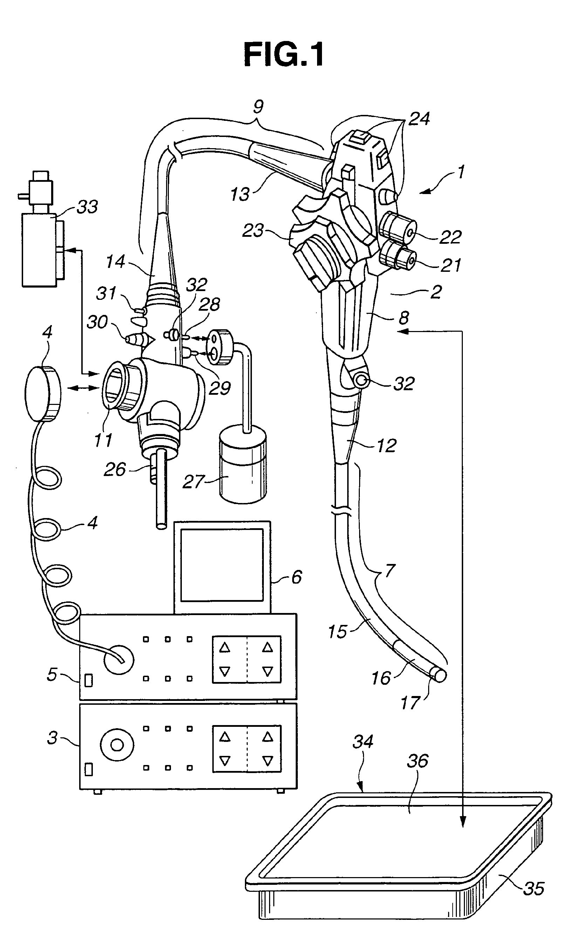

Figs. 1 through 3 show an embodiment of the present invention.

As shown in Fig. 1, an endoscope apparatus 1 according to the present embodiment comprises an endoscope 2 including an unshown image pick-up means, a light source device 3, which is detachably connected to the endoscope 2, for supplying illumination light to a light guide which is inserted into the endoscope 2, a video processor 5, which is connected to the endoscope 2 through a signal cable 4, for controlling the image pick-up means of the endoscope 2, and performing processing for the signals obtained from the image pick-up means so as to output standard format video signals, and a monitor 6 for inputting video signals from the video processor 5 and displaying the endoscope images. Note that the endoscope 2 has a configuration wherein autoclave sterilization (high-temperature and high-pressure steam sterilization) can be performed following washing after use of observation or treatment.

The endoscope 2 principally comprises a fine and elongated and flexible inserting portion 7 and an operating portion 8 included on the base side of the inserting portion 7.

The endoscope 2 includes a flexible universal code 9 extending from the side of the operating portion 8. The universal code 9 includes a connector 10 on the end of the universal code 9, which can be detachably connected to the light source device 3. Furthermore, the connector 10 includes an electric connector 11 on the side thereof. The electric connector 11 is connected to the signal cable 4 which can be connected to the video processor 5.

Furthermore, an inserting-portion bending restriction member 12, an operating-portion bending restriction member 13, and a connector-portion bending restriction member 14, are provided to the connection portion between the inserting portion 7 and the operating portion 8, the connection portion between the operating portion 8 and the universal code 9, and the connection portion between the universal code 9 and the connector 10, respectively, for restricting severe bending of these connection portions.

The inserting portion 7 comprises a flexible tube 15 having flexibility, a bendable curving portion 16 which is provided on the tip side of the flexible tube 15, and a tip portion 17, which is provided to the tip thereof, including an unshown observation optical system, illumination optical system, and the like.

The operating portion 8 includes an air/water supply operating button 21 for operating air supply and water supply, a suctioning operating button 22 for operating suctioning operation, a curving operating knob 23 for operating curving operation for the curving portion 16, multiple remote switches 24 for performing remote control operation of the video processor 5, and a treatment tool inserting opening 25 which is an opening communicating with a treatment tool inserting channel.

The tip portion 17 includes an unshown liquid-supply opening and air/water supply nozzle for discharging a washing liquid or air toward an unshown observation window according to air/water supply operation, and an unshown suctioning opening, which is an opening on the tip side of the unshown treatment tool cannel, for inserting a treatment tool into the inserting portion 7 or suctioning liquid within the body cavity.

The connector 10 includes an air supply cap 26 which is detachably connected to an unshown air supply source incorporated in the light source device 3, a water-supply tank pressurizing cap 28 and a liquid-supply cap 29, which are detachably connected to a water-supply tank 27 which is a liquid supply source, a suctioning cap 30 which is connected to an unshown suctioning source for performing suctioning through the suctioning opening of the tip portion 17, and a water-supply opening cap 31 which is connected to unshown water-supply means for performing water supply through the liquid-supply opening of the tip portion 17. Furthermore, the connector 10 includes a ground terminal cap 32 for returning leakage current to a high-frequency processing device in the event that high-frequency leakage current occurs in the endoscope at the time of performing high-frequency processing or the like.

The electric connector 11 includes an unshown vent portion for communicating between the inside and the outside of the endoscope 2. Also, the electric connector 11 can be detachably connected to a waterproof cap 33 with a pressure regulating valve. Note that the waterproof cap 33 includes an unshown pressure regulating valve. The pressure regulating valve has a configuration wherein in the event that the pressure in the inside of the endoscope exceeds a predetermined value as compared with the pressure in the outside thereof, the pressure regulating valve is released so as to maintain the pressure in the inside of the endoscope in the range of the predetermined pressure. With the present embodiment, the pressure regulating valve has a configuration wherein the aforementioned predetermined value can be set so that the force, which extends the flexible tube 15, caused by the difference in the pressure between the inside of the endoscope and the outside thereof in the event that the pressure in the inside of the endoscope is smaller than the pressure in the outside thereof, is generally the same as the force which contracts the flexible tube 15.

Note that the pressure regulating valve according to the present embodiment has a configuration wherein the pressure regulating valve is released in the event that the difference in pressure between the inside and the outside of the endoscope reaches 15 kPa or more.

On the other hand, at the time of autoclave sterilization, the endoscope 2 is stored in a sterilization storage casing (which will be referred to as "storage casing" hereafter) 34.

The storage casing 34 comprises a tray 35 for storing the endoscope 2, and a back-lid member 36 for the tray 35. The tray 35 and the back-lid member 36 include multiple unshown vents so as to make steam pass through these vents at the time of autoclave sterilization (high-temperature and high-pressure steam sterilization).

The tray 35 includes a fitting portion (not shown) for storing the endoscope 2. The fitting portion is formed in a shape so as to store each component of the endoscope 2 at a predetermined position, and accordingly, upon storing the endoscope in the fitting portion, the endoscope is stored at a predetermined portion. Furthermore, the fitting portion includes an inserting-portion fitting portion (not shown) for storing the inserting portion 7 having flexibility.

Note that for the typical condition for the high-temperature and high-pressure steam sterilization, the conditions stipulated by ST37-1992 of the American National Standards Institute and Association for the Advancement of Medical Instrumentation (ANSI/AAMI) are known, wherein in a case of a pre-vacuum type, the sterilization step is performed for four minutes under 132°C, and in a case of a gravity type, the sterilization step is performed for ten minutes under 132°C.

While the temperature for the sterilization step of the high-temperature and high-pressure steam sterilization is set according to the type of the high-temperature and high-pressure steam sterilization apparatus and the time period of the sterilization step, in general the temperature is set to around in the range of 115°C to 138°C. There are also sterilization apparatuses wherein the temperature for sterilization can be set to around 142°C.

On the other hand, while the time period for the sterilization step is set according to the temperature condition for the sterilization step, in general, the time period is set in the range of around 3 to 60 minutes. There are sterilization apparatuses wherein the time period for sterilization can be set to around 100 minutes.

In general, the processing is performed in the sterilization chamber under a pressure greater than the atmosphere by around +0.2 MPa.

In general, a pre-vacuum type high-temperature and high-pressure steam sterilization process includes a pre-vacuum step for decompressing the sterilization chamber storing a device which is to be subjected to sterilization prior to a sterilization step, and the sterilization step for performing sterilization by supplying the high-pressure and high-temperature steam into the sterilization chamber following the pre-vacuum step.

The pre-vacuum step is performed for facilitating penetration of steam into the fine structures of the device to be sterilized in the following sterilization step. That is to say, the high-pressure and high-temperature steam reaches all parts of the entire device which is to be subjected to sterilization by decompressing the sterilization chamber. In general, the pre-vacuum step is performed in the sterilization chamber under a pressure less than the atmosphere by around -0.07 MPa to -0.09 MPa.

Furthermore, a sterilization process is known which includes a dry step for drying the device which is to be subjected to sterilization by decompressing the sterilization chamber again following the sterilization step. That is to say, in this step, the steam is removed from the sterilization chamber by decompressing the sterilization chamber, thereby prompting drying of the device sterilized in the sterilization chamber. In general, this step is performed in the sterilization chamber under a pressure less than the atmosphere by around -0.07 to -0.09 MPa.

At the time of performing autoclave sterilization (high-temperature and high-pressure steam sterilization) for the endoscope 2, the processing is performed for the endoscope 2 with the waterproof cap 33, including the pressure regulating valve, mounted to the electric connector 11. In this state, the unshown pressure regulating valve of the water-proof valve 33 is closed, and accordingly, the vent opening is closed with the water-proof cap 33, whereby the inside of the endoscope 2 is sealed off from the outside in a watertight manner.

In a case of a sterilization method including the pre-vacuum step, upon the pressure difference occurring wherein the pressure in the outside of the endoscope 2 becomes smaller than the pressure in the inside thereof due to reduction of the pressure in the sterilization chamber in the pre-vacuum step, the pressure regulating valve is released (opened) so as to communicate between the inside of the endoscope 2 and the outside thereof through the vent opening, thereby preventing great pressure difference between the inside of the endoscope 2 and the inside of the sterilization chamber. This eliminates the problem that the endoscope 2 might be damaged due to the pressure difference between the inside and the outside of the endoscope 2 in the pre-vacuum step.

In the sterilization step, the inside of the sterilization chamber is pressurized. As a result, upon the pressure difference occurring wherein the pressure in the outside of the endoscope 2 becomes greater than the pressure in the inside thereof, and the pressure regulating valve is closed. Thus, a great amount of the high-pressure and high-temperature steam does not enter the inside of the endoscope 2 through the water-proof cap 33 and the vent opening.

However, the high-temperature and high-pressure steam gradually penetrates into the inside of the endoscope 2 through the surface of the flexible tube formed of a polymeric material, an O-ring formed of fluororubber or silicone rubber or the like, serving as sealing means provided to the connection portion of the casing of the endoscope 2, and so forth. Note that the endoscope 2 is subjected to the positive pressure from the outside to the inside due to decompressing in the casing in the pre-vacuum step and the pressurizing in the sterilization step.

In a case of a method including a decompressing step after the sterilization step, the pressure in the sterilization chamber is reduced in the decompressing step, whereby the pressure difference occurs wherein the pressure in the outside of the endoscope 2 becomes smaller than the pressure in the inside thereof. In this case, the pressure regulating valve is released (opened) generally at the same time, and accordingly, the insides of the endoscope communicates with the outside thereof through the vent opening, thereby preventing occurrence of greater pressure difference between the inside of the endoscope and the inside of the sterilization chamber. This eliminates the problem that the endoscope 2 might be damaged due to the pressure difference between the inside and the outside of the endoscope 2 in the decompressing step.

Following the decompressing step, the inside of the sterilization chamber is pressurized. As a result, upon the pressure difference occurs wherein the pressure in the outside of the endoscope becomes greater than the pressure in the inside thereof, the pressure regulating valve provided to the water-proof cap 33 is closed.

As described above, at the time of all the steps for the autoclave sterilization (high-temperature and high-pressure steam sterilization) ending, the endoscope 2 is subjected to the positive pressure from the outside to the inside due to reduction of the pressure in the casing of the endoscope 2 in the decompressing step.

Subsequently, upon the water-proof cap 33 being detached from the electric connector 11, the inside of the endoscope communicates with the outside thereof through the vent opening. As a result, the pressure in the inside of the endoscope becomes the same as the atmosphere, and accordingly, the pressure load on the casing of the endoscope becomes zero.

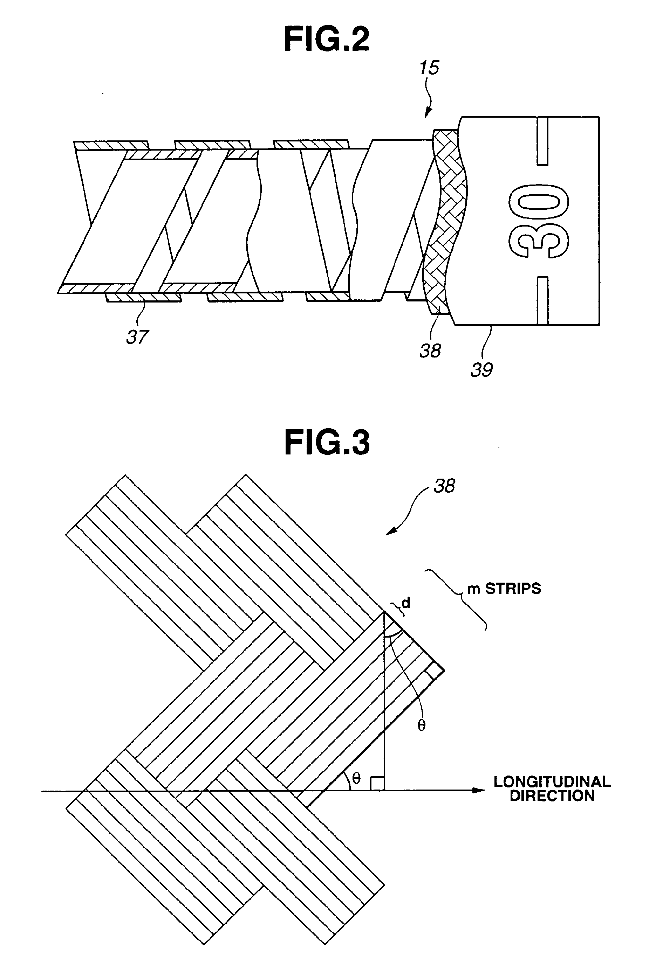

Next, description will be made regarding the configuration of the flexible tube 15 with reference to Figs. 2 and 3.

As shown in Fig. 2, the flexible tube 15 comprises a spiral tube 37 formed of metal strips wound in a spiral shape, a mesh tube 38 in the shape of a mesh for covering the spiral tube 37, and a covering tube 39, which serves as a contracting member or means for generating contraction force, for covering the outer circumference of the mesh tube 38. The flexible tube 15 is formed of the aforementioned components in that order from the inside thereof.

The mesh tube 38 is formed of at least two metal wires in the shape of a mesh, wound at an angle such that the metal wires are not disposed in the direction orthogonal to or parallel to the longitudinal direction thereof.

In the event that the pressure in the inside of the endoscope becomes smaller than the pressure in the outside thereof, the mesh angle of the mesh tube 38 changes to reduce the volume of the inside of the flexible tube 15, due to the pressure difference between the inside and the outside of the endoscope.

At this time, the mesh tube 38 is subjected to the force causing the metal wires to be moved in the longitudinal direction (the direction wherein the metal wires cross with a small angle). That is to say, the mesh tube 38 reduces the diameter of the flexible tube 15, as well as generating the force F1 causing extension of the entire flexible tube in the longitudinal direction thereof.

Note that the force F1 causing the extension of the flexible tube 15 is generated due to the pressure difference on the entire outer circumference of the flexible tube 15, and accordingly, the force F1 is dependent upon the diameter and the length of the flexible tube 15, even under the same pressure.

The present embodiment has a configuration wherein the flexible tube 15 generates the contraction force in the longitudinal direction thereof against the extension force of the flexible tube 15, generally with the same magnitude as the extension force of the flexible tube 15 in the longitudinal direction thereof, due to contraction of the covering tube 39 of the flexible tube 15, thereby suppressing the change in the length of the flexible tube 15.

That is to say, with the present embodiment the covering tube 39 is formed of a resin obtained by blending a styrene resin and an ester resin, which are high polymer materials, at a ratio of 1 to 2. Note that this resin may be formed using a blend of an olefin resin, an amide resin, or the like. Or, any one of a styrene resin, an ester resin, an olefin resin, and an amide resin, may be used independently.

The thickness of the resin is formed so as to match the specifications of the flexible tube portion 15. For example, with the present embodiment, the flexible tube portion 15 is formed 1600 mm in length and 12.9 mm external diameter, with the covering tube 39 formed to a thickness of 0.2 mm and covering the mesh tube 38.

Forming the covering tube 39 by blending a styrene resin and an ester resin, creates a force F2 which contracts under the heat of the autoclave sterilization. This contraction force F2 is determined by the thermal contraction coefficients of each material and the blend ratio thereof, the length of the outer diameter of the flexible tube portion 15, and the thickness of the covering tube.

The covering tube 39 is formed so as to generate the force generally matching the force F1 causing extension of the flexible tube 15.

The endoscope 2 including the flexible tube 15 having such a configuration is subjected to washing and disinfecting following endoscope examination. Following the washing and disinfecting, the endoscope 2 is stored in the storage case 34 and stored in the autoclave sterilization apparatus along with the storage case 34, and is subjected to the above-described autoclave sterilization (high-temperature and high-pressure steam sterilization).

Let us say that the endoscope 2 includes the flexible tube 15 formed with a length of 1600 mm, an outer diameter of 12.9 mm, and a tube-thickness of the covering tube 39 of 0.2 mm, as described above. Furthermore, let us say that the pressure regulating valve provided to the water-proof cap 33 is set so as to be released at the time of the pressure difference reaching 15 kPa or more.

Furthermore, let us say that each step of the autoclave sterilization process is performed with the autoclave sterilization apparatus under the following pressure, for example.

Pre-vacuum step: performed under a pressure less than the atmosphere by 76 kPa.

Sterilization step: performed under a pressure greater than the atmosphere by 216 kPa.

As described above, following all the steps for the autoclave sterilization (high-temperature and high-pressure steam sterilization) being completed, the flexible tube 15, which is a casing member of the endoscope 2, is subjected to the positive pressure from the outside to the inside thereof due to reduction of the pressure in the pre-vacuum step and the increasing pressure in the sterilization step.

Accordingly, a pressure difference of 277 kPa occurs between the inside and outside of the endoscope 2 during the sterilization step, according to the pressure settings for the above-described autoclave apparatus and the settings for releasing pressure with the pressure regulating valve.

As described above, the pressure of 277 kPa occurs, which causes the mesh angle of the mesh tube 38 to be changed and the volume of the inside thereof to be reduced, and accordingly, the mesh tube 38 generates the force F1 causing the diameter of the flexible tube 15 to be reduced and causing extension of the entire flexible tube 15 in the longitudinal direction.

On the other hand, as described above, the covering tube 39 of the flexible tube 15 is formed of a blend of styrene resin and ester resin, and accordingly, the contraction force F2 occurs due to heating in the autoclave sterilization, thereby generating the force F2 generally matches the force F1 causing extension of the flexible tube 15 on the circumference where the covering tube 39 is in contact with the mesh tube 38.

That is to say, the force F1 causing extension of the flexible tube 15 is generally cancelled out by the force F2 causing contraction of the flexible tube 15, thereby suppressing the change in the length of the flexible tube 15 of the endoscope 2 to within ±5% when performing autoclave sterilization.

Thus, with the endoscope 2 according to the present embodiment, the change in the length of the flexible tube 15 due to the autoclave sterilization can be suppressed to within 5%.

Accordingly, if the change in the length of the flexible tube 15 is suppressed to within 5%, the endoscope 2 can prevent the length of incorporated member from becoming short and also can prevent user's wrong recognition of the inserting length.

While an embodiment of the present invention has been described, the present invention is by no means restricted to the above-described embodiment; rather, various modifications may be made without departing from the spirit and scope of the present invention.

### Industrial Applicability

According to the present invention as described above, a flexible tube of an endoscope can be provided wherein change in length during autoclave sterilization can be suppressed.

## Claims

1. A flexible tube of an endoscope having, a mesh tube formed by including a mesh of at least two or more metal wires in directions neither orthogonal nor parallel to the longitudinal direction of an insertion portion;
wherein a contracting member is provided which generates a contraction force in the longitudinal direction, against and approximately equal to a stretching force in the longitudinal direction which is generated in the event that a pressure difference occurs between the inside and outside of the endoscope.

2. A flexible tube of an endoscope according to Claim 1, wherein the contracting member covers the outer circumference of the mesh tube and makes up the covering tube where contraction force is generated during autoclave sterilizing.

3. A flexible tube of an endoscope according to Claim 2, wherein the covering tube is formed of a high polymer material.

4. A flexible tube of an endoscope according to Claim 2, wherein the high polymer material independently uses any one of an ester resin, a styrene resin, an olefin resin, or an amide resin, or uses a blend of any thereof.
